(19) Europäisches Patentamt
European Patent Office
Office européen des brevets

(11) EP 0 580 620 B1

(12) **EUROPÄISCHE PATENTSCHRIFT**

(45) Veröffentlichungstag und Bekanntmachung des Hinweises auf die Patenterteilung:
**18.10.2000 Patentblatt 2000/42**

(21) Anmeldenummer: **92907091.0**

(22) Anmeldetag: **19.03.1992**

(51) Int. Cl.$^7$: **B01F 1/00**

(86) Internationale Anmeldenummer:
**PCT/DE92/00231**

(87) Internationale Veröffentlichungsnummer:
**WO 92/18227 (29.10.1992 Gazette 1992/27)**

# (54) STABILE MULTIPLE EMULSIONEN

STABLE MULTIPLE EMULSIONS

EMULSIONS MULTIPLES STABLES

(84) Benannte Vertragsstaaten:
**AT BE CH DE DK ES FR GB GR IT LI NL SE**

(30) Priorität: **13.04.1991 DE 4112127**
**24.09.1991 DE 4131678**

(43) Veröffentlichungstag der Anmeldung:
**02.02.1994 Patentblatt 1994/05**

(73) Patentinhaber:
**Beiersdorf Aktiengesellschaft**
**20245 Hamburg (DE)**

(72) Erfinder:
- **DAHMS, Gerd**
  **D-5620 Velbert (DE)**
- **GOHLA, Sven, H.**
  **D-2000 Hamburg 52 (DE)**
- **KRÖPKE, Rainer**
  **D-2095 Marschacht 1 (DE)**
- **NIELSEN, Jens**
  **D-2359 Henstedt-Ulzburg (DE)**

(56) Entgegenhaltungen:
**EP-A- 0 166 946** **EP-A- 0 174 377**
**EP-A- 0 391 124** **FR-A- 2 326 914**

- **DATABASE WPIL Week 4185, Derwent Publications Ltd., London, GB; AN 85-252689**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7 (HRS)/3.6

## Beschreibung

Die vorliegende Erfindung betrifft stabile multiple Emulsionen, Verfahren zu ihrer Herstellung sowie ihre Verwendung für kosmetische und medizinische Zwecke.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist bekannt, daß sich multiple Emulsionen - unter anderem - durch eine besonders feine Emulsionstextur auszeichnen können. Diese Eigenschaft eignet sie hervorragend als Basis sowohl für kosmetische wie für medizinische topische Zubereitungen. Wo allerdings Kosmetika nur äußerlich angewandt werden, sind bei medizinischer Verwendung von Emulsionen alle üblichen Applikationsarten, z.B. orale Verabreichungsformen, denkbar.

In einer einfachen Emulsion liegen in der einen Phase feindisperse, von einer Emulgatorhülle umschlossene Tröpfchen der zweiten Phase (Wassertröpfchen in W/O- oder Lipidvesikel in O/W-Emulsionen) vor. In einer multiplen Emulsion (zweiten Grades) hingegen sind in solchen Tröpfchen feiner disperse Tröpfchen der ersten Phase emulgiert. Auch in diesen Tröpfchen wiederum können noch feiner disperse Tröpfchen vorliegen (multiple Emulsion dritten Grades) und so fort.

So wie man also bei den einfachen Emulsionen von W/O- oder O/W-Emulsionen spricht (Wasser-in-Oel oder Oel-in-Wasser), gibt es bei multiplen Emulsionen W/O/W-, O/W/O-, O/W/O/W-, W/O/W/O-Emulsionen und so fort. Multiple Emulsionen, bei welchen die jeweiligen inneren und äußeren Wasserphasen oder inneren und äußeren Ölphasen unterschiedlich geartet sind (also z.B. W/O/W'- und O/W/O'-Emulsionen), sind der Präparation durch Zweitopfverfahren zugängig. Solche Emulsionen, in welchen die inneren und äußeren Wasser- bzw.- Ölphasen nicht unterschiedlich geartet sind, sind sowohl durch Ein- als auch durch Zweitopfverfahren erhältlich.

Die multiplen Emulsionen zweiten Grades werden gelegentlich als "bimultiple Systeme", solche dritten Grades als "trimultiple Systeme" usw., bezeichnet (W.Seifriz, "Studies in Emulsions", J.Phys.Chem., 29 (1925) 738 - 749).

Verfahren zur Herstellung multipler Emulsionen sind dem Fachmann an sich geläufig. So gibt es Zweitopfverfahren, in welchen eine einfache Emulsion (z.B. eine W/O-Emulsion) vorgelegt und durch Zugabe einer weiteren Phase (hier: einer Wasserphase) mit einem entsprechenden Emulgator (hier: einem O/W-Emulgator) in eine multiple Emulsion (hier: eine W/O/W-Emulsion) überführt wird.

Eine zweites Verfahren besteht darin, Emulgatorgemische mit einer Ölphase und einer Wasserphase in einem Eintopfverfahren in eine multiple W/O/W-Emulsion zu überführen. Die Emulgatoren werden in der Ölphase gelöst und mit der Wasserphase vereinigt. Voraussetzung für ein solches Verfahren ist, daß die HLB-Werte (HLB = Hydrophil-Lipophil-Balance) der eingesetzten einzelnen Emulgatoren sich deutlich voneinander unterscheiden.

Die Definition für den HLB-Wert ist für Polyolfettsäureester gegeben durch

$$HLB = 20 * ( 1 - S/A)$$

Für eine Gruppe von Emulgatoren, deren hydrophiler Anteil nur aus Ethylenoxideinheiten besteht, gilt die Formel

$$HLB = E/5$$

wobei

5 = Verseifungszahl des Esters,
A = Säurezahl der zurückgewonnen Säure
E = Massenanteil Ethylenoxid (in %) am Gesamtmolekül

bedeuten.

Literatur: "Kosmetik - Entwicklung, Herstellung und Anwendung kosmetischer Mittel"; W.Umbach (Hrsg.), Georg Thieme Verlag 1988.

Hydrophile Emulgatoren (mit hohen HLB-Werten) sind in der Regel O/W-Emulgatoren. Demgemäß sind hydrophobe oder lipophile Emulgatoren (mit niedrigen HLB-Werten) in der Regel W/O-Emulgatoren.

Die Patentschrift US-A-4,931,210 beschreibt ein Verfahren zur Herstellung von W/O/W-Emulsion, wobei als Emulgatoren Polyglycerinpolyricinoleate verwendet werden.

Obwohl also multiple Emulsionen an sich bekannt sind und es durchaus einfache Verfahren zu ihrer Herstellung gibt, hat es dennoch bis heute an solchen Systemen gemangelt, welche mikroskopisch stabil sind. Dies soll bedeuten, daß sich die multiplen Emulsionen des Standes der Technik mit der Zeit in einfache Emulsionen umwandeln, also eine im Sinne der Multiplizität geringe Lagerungsstabilität aufweisen. Dies ist insbesondere nachteilig, als diese Umwandlungsprodukte eine äußerst inhomogene Tröpfchengrößenverteilung haben.

Bestenfalls sind solche Umwandlungsprodukte unschön oder aus kosmetischer Sicht unelegant. Oft ist jedoch mit der inhomogenen Größenverteilung der Tröpfchen auch mangelnde makroskopische Stabilität verbunden, also die Stabilität gegen die Zersetzung in getrennte Phasen.

Auch in dieser Hinsicht ließen die herkömmlichen multiplen Emulsionen stets zu wünschen übrig.

In dieser Patentanmeldung werden daher unter dem Begriff "Stabilität" gleichrangig sowohl mikroskopische als auch makroskopische Stabilität verstanden, sofern nicht anders angezeigt.

Aufgabe der vorliegenden Erfindung war mithin, stabile multiple Emulsionen zur Verfügung zu stellen und die Nachteile der Zubereitungen des Standes der Technik zu beseitigen.

Weiterhin war es die Aufgabe der Erfindung, Verfahren zu entwickeln, die es in einfacher Weise ermöglichen, gezielt multiple Emulsionen mit den erstrebten vorteilhaften Eigenschaften herzustellen.

Es hat sich überraschenderweise gezeigt, und darin liegt die Lösung der Aufgabe, daß stabile multiple Emulsionen vom Typ W/O/W, welche mindestens ein polares Öl enthalten, ferner enthaltend ein Emulgatorgernisch, bestehend aus

- einem ersten, hydrophoben Emulgator (Emulgator A), oder mehreren solchen Emulgatoren im Gemisch, mit folgenden Eigenschaften:
    - er muß bei der Herstellungstemperatur der Emulsion in der Ölphase der Emulsion zu mehr als 0,01 Gew.-%, insbesondere zu mehr als 5,0 Gew.-% in einem unpolaren Öl löslich sein, wenn ein unpolares Öl verwendet wird, oder
    - er muß bei zu mehr als 0,02 Gew.-%, insbesondere zu mehr als 20,0 Gew.-% in dem polaren Öl löslich sein,
    - er darf unterhalb von 50° in Wasser nicht isotrop löslich sein
    - er darf im Temperaturintervall zwischen etwa 50° bis zur Herstellungstemperatur der Emulsion in Wasser isotrop löslich sein
    - er muß im Temperaturintervall zwischen etwa 50° bis zur Herstellungstemperatur der Emulsion primär als W/O-Emulgator fungieren
- und einem zweiten, hydrophilen Emulgator (Emulgator B), oder mehreren solchen Emulgatoren im Gemisch, mit folgenden Eigenschaften:
    - er muß in Wasser isotrop löslich sein,
    - er muß im Temperaturintervall zwischen etwa 50° bis zur Herstellungstemperatur der Emulsion eine stabile Löslichkeit aufweisen,

den Nachteilen des Standes der Technik abhelfen.

Die Europäische Patentanmeldung 391 124 beschreibt multiple Emulsionen, welche aber vom O/W/O-Typ sind.

Die Japanische Patentanmeldung Sho-60/166604, ausgewiesen als Derwent-Patentabstract, Zugriffsnummer 85-252689, betrifft ebenso W/O/W-Emulsionen, wie das französische Patent 2 326 914.

Vorteilhaft werden die Emulgatoren so gewählt, daß der hydrophile Emulgator den hydrophoben Emulgator am Phaseninversionspunkt benetzt, also als Netzmittel für die ölige Phase wirkt und das Entstehen einer in-situ-multiplen Emulsion in einem Einschrittverfahren erst möglich macht.

Von Vorteil ist ferner, wenn der Emulgator A und der Emulgator B eine gemeinsame flüssigkristalline Phase bilden können, welche die Grenzschicht der Emulsionströpfchen formt.

Insbesondere vorteilhaft sind Emulgatorengemische, bei welchen der oder die hydrophoben Emulgatoren A bei der Verarbeitungstemperatur, typischerweise etwa 70°C, zu mehr als 0,01 Gew.-%, vorteilhaft zu mehr als 5,0 Gew.-

% in einem unpolaren Öl löslich sind, wenn ein unpolares Öl verwendet wird, oder bei welchen der oder die Emulgatoren A zu mehr als 0,02 Gew.-%, vorteilhaft zu mehr als 20,0 Gew.-% in einem polaren Öl löslich sind, wenn ein polares Öl verwendet wird.

Dabei können im Einzelfalle die vorteilhaften Konzentrationen des Emulgators A in einem unpolaren Öl von 5,0 Gew.-% abweichen, wenn die kritische Micellbildungskonzentration (CMC) in diesem Öl deutlich von den als Richtwert angenommenen 5,0 Gew.-% abweichen. In polaren Ölen wird eine kritische Micellbildungskonzentration nicht beobachtet.

Unpolare Öle sind beispielsweise Mineralöle, also solche, die sich durch einen niedrigen Gehalt an Bestandteilen mit polaren Molekülgruppen auszeichnen.

Polare Öle sind beispielsweise vegetabile Öle (z.B. Mais- und Weizenkeimöl) bzw. deren synthetische und teilsynthetische Äquivalente und ähnliche Öle, also solche, die sich durch einen höheren Gehalt an Bestandteilen mit polaren Molekülgruppen, zum Beispiel Ester- oder Ethergruppen, auszeichnen.

In dieser Patentanmeldung wird unterschieden zwischen isotroper Löslichkeit und anisotroper Löslichkeit, da Tenside mit den herkömmlichen Begriffen der Löslichkeit nur schwer zu erfassen sind. Ein Stoff ist in einem anderen Stoff isotrop lösliCh, wenn er ohne Micellenbildung oder ohne andere mit lichtmikroskopischen Verfahren nachweisbare Aggregatbildung in diesem Stoff eine echte Lösung bildet. Ein Stoff ist in einem anderen anisotrop löslich, wenn er unter Micellenbildung oder unter Bildung anderer mit lichtmikroskopischen Verfahren nachweisbarer Aggregate in diesem Stoff löslich, beispielsweise kolloidal löslich ist.

Die hydrophoben Emulgatoren A können günstig gewählt werden aus der Gruppe der

Glycerinether, beispielsweise

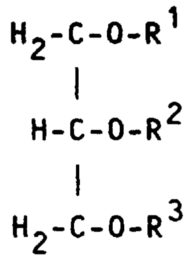

und Polyglycerinether, wobei die Reste $R^{1-5}$ H, oder einen gesättigten oder ungesättigten organischen Rest darstellen,

Polyglycerinester,

z.B. 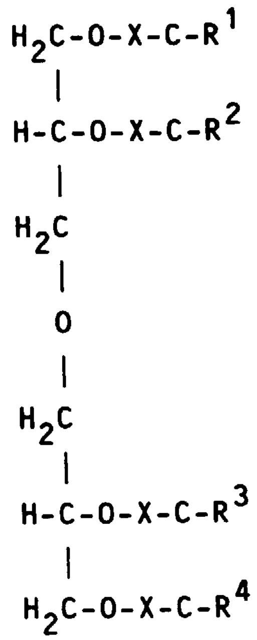 (Diglycerylester),

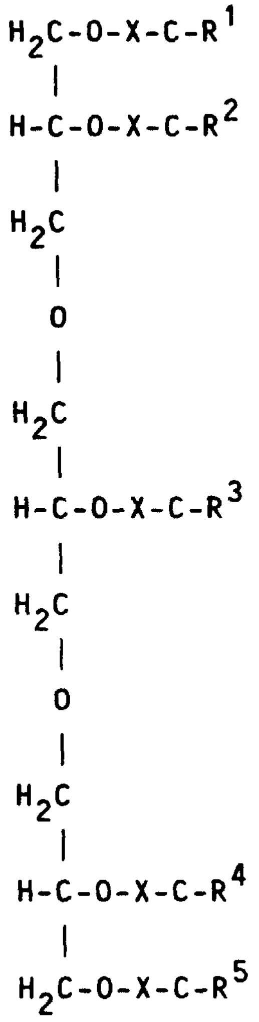

(Triglycerylester),

wobei $R^{1-5}$ , wie der Fachmann weiß, unabhängig voneinander H, oder einen gesättigten oder ungesättigten organischen Rest darstellen können und X eine Einfachbindung oder einen Carbonylrest darstellen kann.

sowie Methylglucoside, Saccharoseester, Alkylpolyglycoside, Sorbitanester, Sorbitolester, Isosorbidester. Siehe zu den vorstehend genannten Emulgatorenklassen das "Lexikon der Hilfsstoffe für Pharmazie, Kosmetik und angrenzende Gebiete" (H.P.Fiedler, Editio Cantor Aulendorf, Dritte Auflage, 1989) zu den entsprechenden Stichwörtern, sowie ggf. Parallel- und Querverweise. Die Offenbarung in diesem Nachschlagewerk zu diesen Stichwörtern wird in die Offenbarung dieser Patentanmeldung einbezogen.

Es ist günstig, Vertreter der vorgenannten Emulgatorenklassen zu wählen, die, wenn sie als Ester vorliegen, vorwiegend gesättigte Fettsäurereste enthalten, bzw, wenn sie als Ether vorliegen, von gesättigten Fettalkoholen abgeleitet sind, das bedeutet also beispielsweise für die vorstehend exemplarisch aufgeführten Glycerinether und Polyglycerinester, daß, wenn $R^{1-5}$ nicht einen Wasserstoffrest darstellen, sie gesättigten Kohlenwasserstoffreste darstellen soll. Das gleiche gilt für die anderen vorgenannten Emulgatorenklassen.

Besonders günstig sind solche hydrophoben Emulgatoren zu verwenden, welche bei Raumtemperatur in fester Form vorliegen.

Die hydrophoben Emulgatoren werden besonders günstig gewählt aus der Gruppe, bestehend aus

Sorbitanoleat, Sorbitanmonostearat, Steareth-2, Glycerinmonostearat, Glyceryloleat, Polyglycerin-4-isostearat, PEG-7-hydriertes Ricinusöl, PEG-40-Sorbitanmonostearat, Laureth-4.

Der oder die Emulgatoren 8 können ebenfalls vorteilhaft aus diesen Substanzen gewählt werden, wobei nur darauf zu achten ist, daß der oder die Emulgatoren B über die eingangs geforderten Löslichkeitseigenschaften verfügen.

Die hydrophilen Emulgatoren B werden besonders günstig gewählt aus der Gruppe, bestehend aus

Oleth-20, PEG-40-Stearat, Sucrosecocoat, Trilaureth-4-phosphat, Gluceth-20, PPG-20-Methylglucoseether, Polyglycerin-3-stearat, Polysorbat-120, Sucroselaurat, Ceteareth 20.

Eine vorteilhafte Verkörperung der vorliegenden Erfindung besteht ferner auch darin, und stabile multiple Emulsionen werden auch dann erfindungsgemäß erhalten, wenn ein Emulgatorengemisch verwendet wird, bei dem der hydrophobe Emulgator A und der hydrophile Emulgator B ethoxylierte Produkte darstellen. Erfindungsgemäß ist also ein Emulgatorengemisch, bestehend aus

(a) mindestens einem Emulgator der allgemeinen Formel

$$R^1-Q-O-(\underset{X}{\overset{H}{C}}-\underset{Y}{\overset{H}{C}}-Z-O-)_m-H \qquad \text{(Emulgator A)},$$

wobei

| | |
|---|---|
| $R^1$ = | $C_{10-30}$-Alkyl |
| Q = | Methylen oder Carbonyl |
| X = | H oder Methyl |
| Y = | H oder Methyl |
| Z = | Methylen oder eine Einfachbindung |
| m = | eine Zahl von 1 bis 40 |

darstellen,
und

(b) mindestens einem Emulgator der allgemeinen Formel

$$R^2-Q-O-(\underset{X}{\overset{H}{C}}-\underset{Y}{\overset{H}{C}}-Z-O-)_n-H \qquad \text{(Emulgator B)},$$

wobei

| | |
|---|---|
| $R^2$ = | $C_{10-30}$-Alkyl |
| Q = | Methylen oder Carbonyl |
| X = | H oder Methyl |
| Y = | H oder Methyl |
| Z = | Methylen oder eine Einfachbindung |
| n = | eine Zahl von 10 bis 400 |

darstellen, mit der Maßgabe, daß der Quotient n/m wenigstens 1,5 betragen muß,
sowie

(c) gegebenenfalls weitere Emulgatoren.

Vorteilhaft können der oder die Emulgatoren A aus der Gruppe der Polyoxyethylenfettalkoholether gewählt werden; bevorzugt haben diese Emulgatoren dann die allgemeine Formel

$$R^1\text{-}Q\text{-}O\text{-}(\underset{X}{\overset{H}{C}}\text{-}\underset{Y}{\overset{H}{C}}\text{-}Z\text{-}O\text{-})_m\text{-}H \qquad \text{(Emulgator A),}$$

wobei

| | |
|---|---|
| $R^1$ = | $C_{10-30}$-Alkyl |
| Q = | Methylen |
| X = | H |
| Y = | H |
| Z = | eine Einfachbindung |
| m = | eine Zahl von 1 bis 20 |

darstellen.

Besonders bevorzugt sind dann solche Emulgatoren A, bei denen

| | |
|---|---|
| $R^1$ = | $C_{15-25}$-Alkyl |
| Q = | Methylen |
| X = | H |
| Y = | H |
| Z = | eine Einfachbindung |
| m = | eine Zahl von 1 bis 10 |

bedeuten.

Vorteilhaft können der oder die Emulgatoren A aber auch gewählt werden aus der Gruppe der Polyoxyethylenfettsäureester; bevorzugt haben diese Emulgatoren dann die allgemeine Formel

$$R^1\text{-}Q\text{-}O\text{-}(\underset{X}{\overset{H}{C}}\text{-}\underset{Y}{\overset{H}{C}}\text{-}Z\text{-}O\text{-})_m\text{-}H \qquad \text{(Emulgator A),}$$

wobei

| | |
|---|---|
| $R^1$ = | $C_{10-30}$-Alkyl |
| Q = | Carbonyl |
| X = | H |
| Y = | H |
| Z = | eine Einfachbindung |
| m = | eine Zahl von 1 bis 20 |

darstellen.

Besonders bevorzugt sind dann solche Emulgatoren A, bei denen

| | |
|---|---|
| $R^1$ = | $C_{15-25}$-Alkyl |
| Q = | Carbonyl |
| X = | H |
| Y = | H |
| Z = | eine Einfachbindung |
| m = | eine Zahl von 1 bis 10 |

bedeuten.

Bevorzugt wird der oder werden die Emulgatoren B gewählt aus der Gruppe der Polyoxyethylenfettalkoholether; bevorzugt haben diese Emulgatoren dann die allgemeine Formel

$$R^2\text{-Q-O-}(\overset{H}{\underset{X}{C}}\text{-}\overset{H}{\underset{Y}{C}}\text{-Z-O-})_n\text{-H} \qquad \text{(Emulgator B)},$$

wobei

$R^2$ = $C_{10\text{-}30}$-Alkyl
Q = Methylen
X = H
Y = H
Z = eine Einfachbindung
n = eine Zahl von 10 bis 400

darstellen.

Besonders bevorzugt sind dann solche Emulgatoren B, bei denen

$R^2$ = $C_{15\text{-}25}$-Alkyl
Q = Methylen
X = H
Y = H
Z = eine Einfachbindung
n = eine Zahl von 10 bis 400

bedeuten.

Vorteilhaft können der oder die Emulgatoren B aber auch gewählt werden aus der Gruppe der Polyoxyethylenfettsäureester; bevorzugt haben diese Emulgatoren dann die allgemeine Formel

$$R^2\text{-Q-O-}(\overset{H}{\underset{X}{C}}\text{-}\overset{H}{\underset{Y}{C}}\text{-Z-O-})_n\text{-H} \qquad \text{(Emulgator B)},$$

wobei

$R^2$ = $C_{10\text{-}30}$-Alkyl
Q = Carbonyl
X = H
Y = H
Z = eine Einfachbindung
m = eine Zahl von 10 bis 400

darstellen.

Besonders bevorzugt sind dann solche Emulgatoren B, bei denen

$R^2$ = $C_{15\text{-}25}$-Alkyl
Q = Carbonyl
X = H
Y = H
Z = eine Einfachbindung

n = eine Zahl von 10 bis 400

bedeuten.

Es ist aber auch gegebenenfalls vorteilhaft, solche vorstehend beschriebenen Emulgatoren A, welche keine ethoxylierten Produkte darstellen, mit solchen Emulgatoren B zu mischen, welche ethoxylierte Produkte darstellen, sofern die geforderten physikalischen Eigenschaften der Emulgatoren gegeben sind.

Ferner ist es auch gegebenenfalls vorteilhaft, solche vorstehend beschriebenen Emulgatoren B, welche keine ethoxylierten Produkte darstellen, mit solchen Emulgatoren A zu mischen, welche ethoxylierte Produkte darstellen, sofern die geforderten physikalischen Eigenschaften der Emulgatoren gegeben sind.

Als günstige Zusammensetzungen gemäß der vorliegenden Erfindung werden multiple Emulsionen angesehen, bei denen die Emulgatoren A und/oder B Emulgatoren des Steareth-Typs sind. Steareth-Emulgatoren sind beispielsweise unter der Handelsbezeichnung Brij$^R$ (Atlas-Chemie) erhältlich.

Als besonders günstig hat sich das Emulgatorsystem Steareth-2 (Emulgator A) und Steareth-21 (Emulgator B) herausgestellt.

Steareth-2 ist POE-(2)-Stearylalkohol, also ein mit zwei Ethylenoxideinheiten ethoxylierter Stearylalkohol.

Steareth-21 ist POE-(21)-Stearylalkohol, also ein mit 21 Ethylenoxideinheiten ethoxylierter Stearylalkohol.

Erfindungsgemäß sind also stabile multiple Emulsionen mit einem Gehalt an den vorstehend bezeichneten und in den Ansprüchen näher beschriebenen Emulgatoren, sowie den üblichen kosmetischen oder medizinischen Bestandteilen, sowie gegebenenfalls weiteren Emulgatoren und/oder Hilfs- und/oder Zusatzstoffe.

Bevorzugt liegt die Konzentration der Summe von Emulgator A und B zwischen 0,5 und 15,0 %, bezogen auf die gesamte kosmetische oder medizinische Zusammensetzung.

Die molaren Verhältnisse von Emulgator A : B können vorteilhaft von 10 : 1 bis 1 : 10, besonders vorteilhaft 5 : 1 bis 1 : 5, insbesondere 2 : 1 bis 1 : 2, bevorzugt 3 : 2 bis 2 : 3, ganz besonders bevorzugt 1,2 : 1 bis 1 : 1,2 betragen.

Wenn in dieser Patentanmeldung von Prozentanteilen die Rede ist, sind immer Gewichtsprozente gemeint, sofern nicht ausdrücklich anders vermerkt.

Vorteilhaft wird ein Gemisch von Steareth-2 : Steareth-21 im Verhältnis von 5 : 1 bis 1 : 5 eingesetzt. Die günstigste Gesamtmenge an diesem Emulgatorsystem, bezogen auf die Gesamtzusammensetzung, beträgt in dieser Ausführungsform zwischen 1 und 10 %.

Ganz besonders vorteilhaft sind Zusammensetzungen, welche außer den erfindungsgemäßen Zusammensetzungen noch einen Gehalt an Linolsäureglyceriden (Trivialname für Glycerin-linolsäureester) aufweisen.

Vorteilhaft ist es auch, pflanzliche Öle zu verwenden, deren Gehalt an Linolsäureglyceriden hoch ist.

Insbesondere liegt die Wirkung der pflanzlichen Öle bzw. der Linolsäureglyceride überraschenderweise darin, daß sie die mikroskopische Struktur der erfindungsgemäßen multiplen Emulsionen vorteilhaft beeinflussen, sie führen zur Verkleinerung der multiplen Emulsionströpfchen und zu einer erhöhten Multiplizität der Systeme.

Unter den Linolsäureglyceriden, nachfolgend sind sie in ihrer Fischer-Projektion dargestellt, sind zu verstehen:

Die Glycerin-monolinolsäureester (der Glycerin-1-monolinolsäureester hat zwei optische Isomere):

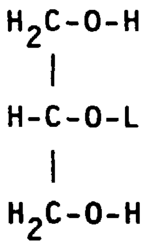

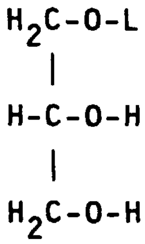

und

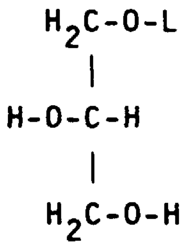

Die Glycerin-dilinolsäureester (der Glycerin-1,2-dilinolsäureester hat zwei optische Isomere):

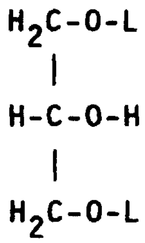

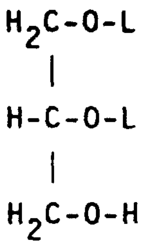

und

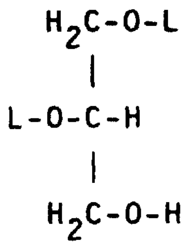

Der Glycerin-trilinolsäureester:

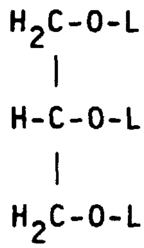

L stellt dabei den cis,cis-9,12-Octadecadienoylrest dar.

Es spielt keine wesentliche Rolle, ob Öle verwendet werden, in welchen die Linolsäureglyceride unterschiedlicher Stöchiometrie und Isomerie als Einzelsubstanzen oder in beliebiger Abmischung untereinander vorliegen.

Die pflanzlichen Öle können bevorzugt gewählt werden aus der Gruppe bestehend aus

Weizenkeimöl, Traubenkernöl, Kukuinosöl, Safloröl und weiteren Ölen, welche mindestens 5 Gew.-% an Linolsäureglyceriden enthalten.

Wo der Durchmesser der multiplen Emulsionen ohne Zusatz dieser Öle typischerweise 20 µm beträgt, kann der Durchmesser der Tröpfchen mit einem solchen Zusatz auf 5 - 10 µm gesenkt werden.

Gleichzeitig erhöhen die pflanzlichen Öle bzw. Linolsäureglyceride die Ausbeute an multiplen Emulsionströpfchen.

Erfindungsgemäß können die multiplen Emulsionen 0,5 - 50 Gew.-% an pflanzlichen Ölen enthalten, vorteilhaft ist, Gehalte von 1,0 - 10,0 Gew.-% dieser Öle zu verwenden.

Die erfindungsgemäßen Zusammensetzungen können ferner einen Gehalt an Stearinsäure und/oder Hydroxyoctacosanyl-hydroxystearat aufweisen, wenn dies gewünscht wird. In überraschender Weise erhöht ein Zusatz an dieser Substanz die thermische Stabilität der erfindungsgemäßen multiplen Emulsionen. Bevorzugt ist, Gehalte von 0,1 - 10 Gew.-% von Stearinsäure und/oder Hydroxyoctacosanyl-hydroxystearat in den erfindungsgemäßen Zusammensetzungen zu verwenden. Vorteilhaft sind besonders Gewichtsverhältnisse von Stearinsäure und/oder Hydroxyoctacosanyl-hydroxystearat von 10 : 1 bis 1 : 10.

Besonders vorteilhaft ist es, pflanzliche Öle und Stearinsäure und/oder Hydroxyoctacosanyl-hydroxystearat gleichzeitig in den erfindungsgemäßen Zusammensetzungen einzusetzen.

Die erfindungsgemäßen multiplen Emulsionen können zwar an sich nach bekannten Verfahren, d.h., sowohl in Einwie auch in Zweitopfverfahren, aus den Ausgangssubstanzen hergestellt werden. Es hat sich aber gezeigt, und darin wird eine weitere vorteilhafte Verkörperung der vorliegenden Erfindung gesehen, daß besonders feintexturierte und hochstabile multiple Emulsionen erhalten werden, wenn nicht, wie bisher üblich, die Gesamtmenge der Emulgatoren in die Fettphase gegeben und hernach mit der Wasserphase vereinigt wird. Stattdessen werden der oder die hydrophilen Emulgatoren (Emulgator B) der Wasserphase und der oder die hydrophoben Emulgatoren (Emulgator A) der Ölphase einverleibt. Beide Phasen werden dann, vorteilhaft bei einer Temperatur, bei welcher die Fettphase deutlich flüssig vorliegt, miteinander vereinigt. Dieses Verfahren ist ein Eintopfverfahren.

Erfindungsgemäß ist also ein Verfahren zur Herstellung stabiler multipler Emulsionen, dadurch gekennzeichnet, daß der hydrophile Emulgator der Wasserphase und der hydrophobe Emulgator der Ölphase einverleibt werden und beide Phasen dann, vorteilhaft bei einer Temperatur, bei welcher die Fettphase deutlich flüssig vorliegt, miteinander vereinigt werden.

Vorteilhafte Hilfs- und Zusatzstoffe sind beispielsweise konsistenzgebende Mittel Füllstoffe, Parfum, Farbstoffe, Emulgatoren, zusätzliche Wirkstoffe wie Vitamine oder Proteine, Lichtschutzmittel, Stabilisatoren, Antioxidantien, Konservierungsmittel, Insektenrepellentien, Alkohol, Wasser, Salze, proteolytisch oder keratolytisch wirksame Substanzen usw. Vorteilhaft können die erfindungsgemäßen multiplen Emulsionen in Pflegeprodukten wie beispielsweise Gesichts-, Hand-, oder Körpercrèmes oder -lotionen, Sonnenschutzformulierungen, Feuchtigkeitscrèmes, Massagecrèmes, Nagelpflegeprodukten und dergleichen eingesetzt werden.

Die nachfolgenden Beispiele sollen die vorliegende Erfindung näher beschreiben, ohne daß jedoch beabsichtigt ist, die Erfindung auf diese Beispiele zu beschränken. Vielmehr ist der Fachmann imstande, die Erfindung durch sein Fachwissen zu variieren, ohne dabei den Boden der Erfindung zu verlassen.

**Beispiel 1**

| Feuchtigkeitscrème | |
|---|---|
| Sorbitanmonostearat | 5,00 % |
| PEG-40-Stearat | 3,00 % |
| Petrolatum DAB 9 | 4,20 % |
| Paraffinöl DAB 9 | 6,00 % |
| Isopropylpalmitat | 2,50 % |
| Cetearylalkohol | 1,00 % |
| Hydroxyoctacosanylhydroxystearat | 0,80 % |
| $MgSO_4$ | 0,60 % |
| Cyclomethicone | 2,50 % |
| Safloröl | 4,00 % |
| Wasser VES | ad 100,00 % |

**Beispiel 2**

| Feuchtigkeitscrème | |
|---|---|
| Steareth-2 | 5,00 % |
| Sucrosecocoat | 3,00 % |
| Petrolatum DAB 9 | 4,20 % |
| Paraffinöl DAB 9 | 6,00 % |
| Isopropylpalmitat | 2,50 % |
| Cetearylalkohol | 1,00 % |
| Hydroxyoctacosanyl hydroxystearat | 0,80 % |
| $MgSO_4$ | 0,60 % |
| Cyclomethicone | 2,50 % |
| Traubenkernöl | 4,50 % |
| Wasser VES | ad 100,00 % |

**Beispiel 3**

| Feuchtigkeitscrème | |
|---|---|
| Glycerinmonostearat | 5,00 % |
| Sucrosecocoat | 3,00 % |
| Petrolatum DAB 9 | 4,20 % |
| Paraffinöl DAB 9 | 6,00 % |
| Isopropylpalmitat | 2,50 % |
| Cetearylalkohol | 1,00 % |
| Hydroxyoctacosanyl-hydroxystearat | 0,80 % |
| $MgSO_4$ | 0,60 % |
| Cyclomethicone | 2,50 % |
| Kukuinußöl | 6,00 % |
| Wasser VES | ad 100,00 % |

**Beispiel 4**

| Feuchtigkeitscrème | |
|---|---|
| Glyceryloleat | 5,00 % |
| Trilaureth-4-phosphat | 3,00 % |
| Petrolatum DAB 9 | 4,20 % |
| Paraffinöl DAB 9 | 6,00 % |
| Isopropylpalmitat | 2,50 % |
| Cetearylalkohol | 1,00 % |
| Hydroxyoctacosanyl-hydroxystearat | 0,80 % |
| $MgSO_4$ | 0,60 % |
| Cyclomethicone | 2,50 % |
| Weizenkeimöl | 7,00 % |
| Wasser VES | ad 100,00 % |

**Beispiel 5**

| Feuchtigkeitscrème | |
|---|---|
| Polyglycerin-4-isostearat | 5,00 % |
| Gluceth-20 | 3,00 % |
| Petrolatum DAB 9 | 4,20 % |
| Paraffinöl DAB 9 | 6,00 % |
| Isopropylpalmitat | 2,50 % |
| Cetearylalkohol | 1,00 % |
| Hydroxyoctacosanyl-hydroxystearat | 0,80 % |
| $MgSO_4$ | 0,60 % |
| Cyclomethicone | 2,50 % |
| Weizenkeimöl | 4,50 % |
| Stearinsäure | 3,00 % |
| Wasser VES | ad 100,00 % |

**Beispiel 6**

| Feuchtigkeitscrème | |
|---|---|
| PEG-7-hydriertes Ricinusöl | 5,00 % |
| PPG-20-Methylglucoseether | 3,00 % |
| Petrolatum DAB 9 | 4,20 % |
| Paraffinöl DAB 9 | 6,00 % |
| Isopropylpalmitat | 2,50 % |
| Cetearylalkohol | 1,00 % |
| Hydroxyoctacosanyl-hydroxystearat | 0,80 % |
| $MgSO_4$ | 0,60 % |
| Cyclomethicone | 2,50 % |
| Kukuinußöl | 5,30 % |
| Stearinsäure | 3,00 % |
| Wasser VES | ad 100,00 % |

**Beispiel 7**

| Feuchtigkeitscrème | |
|---|---|
| PEG-40-Sorbitanmonostearat | 5,00 % |
| Polyglycerin-3-stearat | 3,00 % |
| Petrolatum DAB 9 | 4,20 % |
| Paraffinöl DAB 9 | 6,00 % |
| Isopropylpalmitat | 2,50 % |
| Cetearylalkohol | 1,00 % |
| Hydroxyoctacosanylhydroxystearat | 0,80 % |
| $MgSO_4$ | 0,60 % |
| Cyclomethicone | 2,50 % |
| Traubenkernöl | 4,80 % |
| Stearinsäure | 3,80 % |
| Wasser VES | ad 100,00 % |

**Beispiel 8**

| Handschutzcrème | |
|---|---|
| Laureth-4 | 5,00 % |
| Polysorbat-120 | 3,00 % |
| PEG-40 Stearat | 2,00 % |
| Petrolatum DAB 9 | 1,50 % |
| Paraffinöl DAB 9 | 6,00 % |
| Isopropylpalmitat | 3,00 % |
| $C_{12-15}$-Alkohol-Benzoate | 1,50 % |
| Cetearylalkohol | 1,00 % |
| Hydroxyoctacosanyl-hydroxystearat | 0,40 % |
| $MgSO_4$ | 0,60 % |
| Cyclomethicone | 2,50 % |
| Wasser VES | ad 100,00 % |

**Beispiel 9**

| Körperlotion | |
|---|---|
| Glyceryloleat | 2,50 % |
| Sucroselaurat | 1,00 % |
| PEG-40 Stearat | 0,75 % |
| Petrolatum DAB 9 | 1,50 % |
| Paraffinöl DAB 9 | 6,00 % |
| Isopropylpalmitat | 3,00 % |
| $C_{12-15}$-Alkohol-Benzoate | 1,50 % |
| Cetearylalkohol | 1,00 % |
| Hydroxyoctacosanyl-hydroxystearat | 0,40 % |
| $MgSO_4$ | 0,60 % |
| Cyclomethicone | 2,50 % |
| Wasser VES | ad 100,00 % |

**Beispiel 10**

| Emulsion zur Hand- und Nagelpflege | |
|---|---|
| Laureth-4 | 5,00 % |
| Ceteareth 20 | 3,00 % |
| Petrolatum DAB 9 | 4,20 % |
| Paraffinöl DAB 9 | 6,90 % |
| PPG-15 Stearylether | 4,00 % |
| Cyclomethicon | 2,50 % |
| Konserv.-Mittel | 0,50 % |
| Wasser VES | ad 100,00 % |

**Beispiel 11**

| Feuchtigkeitslotion | |
|---|---|
| Laureth-4 | 5,00 % |
| Ceteareth 20 | 3,00 % |

(fortgesetzt)

| Feuchtigkeitslotion | |
|---|---|
| Petrolatum DAB 9 | 2,20 % |
| Squalan | 4,00 % |
| Isopropylpalmitat | 2,50 % |
| Ethylhexylcocoat | 4,00 % |
| Cetearylalkohol | 1,00 % |
| Hydroxyoctacosanyl-hydroxystearat | 0,80 % |
| $MgSO_4$ | 0,60 % |
| Cyclomethicone | 2,50 % |
| Konserv.-Mittel | 0,50 % |
| Wasser VES | ad 100,00 % |

**Beispiel 12**

| Augenpflegecrème | |
|---|---|
| Steareth-2 | 3,75 % |
| Steareth-21 | 2,25 % |
| Petrolatum DAB 9 | 1,20 % |
| Squalan | 4,00 % |
| Ethylhexylcocoat | 4,00 % |
| Cetearylalkohol | 0,50 % |
| Hydroxyoctacosanyl-hydroxystearat | 0,80 % |
| $MgSO_4$ | 0,60 % |
| Cyclomethicone | 2,50 % |
| Konserv.-Mittel | 0,50 % |
| Wasser VES | ad 100,00 % |

**Beispiel 13**

| Feuchtigkeitslotion | |
|---|---|
| Steareth-2 | 3,75 % |
| Steareth-21 | 2,25 % |
| Petrolatum DAB 9 | 1,20 % |
| Squalan | 4,00 % |
| Ethylhexylcocoat | 4,00 % |

(fortgesetzt)

| Feuchtigkeitslotion | |
|---|---|
| Cetearylalkohol | 0,50 % |
| Schibutter | 4,00 % |
| Hydroxyoctacosanyl-hydroxystearat | 0,80 % |
| $MgSO_4$ | 0,60 % |
| Cyclomethicone | 2,50 % |
| Konserv.-Mittel | 0,50 % |
| Wasser VES | ad 100,00 % |

**Patentansprüche**

1. Stabile multiple Emulsionen vom Typ W/O/W, welche mindestens ein polares Öl enthalten, ferner enthaltend ein Emulgatorgemisch, bestehend aus

   - einem ersten, hydrophoben Emulgator (Emulgator A), oder mehreren solchen Emulgatoren im Gemisch, mit folgenden Eigenschaften:

     - er muß bei der Herstellungstemperatur der Emulsion in der Ölphase der Emulsion zu mehr als 0,01 Gew.-%, insbesondere zu mehr als 5,0 Gew.-% in einem unpolaren Öl löslich sein, wenn ein unpolares Öl verwendet wird, oder
     - er muß bei zu mehr als 0,02 Gew.-%, insbesondere zu mehr als 20,0 Gew.-% in dem polaren Öl löslich sein,
     - er darf unterhalb von 50° in Wasser nicht isotrop löslich sein
     - er darf im Temperaturintervall zwischen etwa 50° bis zur Herstellungstemperatur der Emulsion in Wasser isotrop löslich sein
     - er muß im Temperaturintervall zwischen etwa 50° bis zur Herstellungstemperatur der Emulsion primär als W/O-Emulgator fungieren

   - und einem zweiten, hydrophilen Emulgator (Emulgator B), oder mehreren solchen Emulgatoren im Gemisch, mit folgenden Eigenschaften:

     - er muß in Wasser isotrop löslich sein,
     - er muß im Temperaturintervall zwischen etwa 50° bis zur Herstellungstemperatur der Emulsion eine stabile Löslichkeit aufweisen.

2. Stabile multiple Emulsionen nach Anspruch 1, bei welchen der oder die hydrophoben Emulgatoren A gewählt werden aus der Gruppe der Glycerinether, Polyglycerinether, Polyglycerinester, Methylglucoside, Saccharoseester, Alkylpolyglycoside, Sorbitanester, Sorbitolester, und Isosorbidester.

3. Stabile multiple Emulsionen nach Anspruch 1, bei welchen der oder die hydrophilen Emulgatoren B gewählt werden aus der Gruppe der Glycerinether, Polyglycerinether, Polyglycerinester, Methylglucoside, Saccharoseester, Alkylpolyglycoside, Sorbitanester, Sorbitolester, und Isosorbidester.

4. Stabile multiple Emulsionen nach Anspruch 1, bei welchen der oder die hydrophoben Emulgatoren A gewählt werden aus der Gruppe Sorbitanoleat, Sorbitanmonostearat, Steareth-2, Glycerinmonostearat, Glyceryloleat, Polyglycerin-4-isostearat, PEG-7-hydriertes Ricinusöl, PEG-40-Sorbitanmonostearat, Laureth-4 und die hydrophilen Emulgatoren B gewählt werden aus der Gruppe Oleth-20, PEG-40-Stearat, Sucrosecocoat, Trilaureth-4-phosphat, Gluceth-20, PPG-20-Methylglucoseether, Polyglycerin-3-stearat, Polysorbat-120, Sucroselaurat, Ceteareth 20.

5. Stabile multiple Emulsionen nach Anspruch 1, enthaltend

   (a) mindestens einen hydrophoben Emulgator der allgemeinen Formel

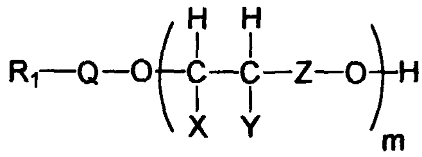

(Emulgator A),

wobei

$R_1$ = $C_{10-30}$-Alkyl
Q = Methylen oder Carbonyl
X = H oder Methyl
Y = H oder Methyl
Z = Methylen oder eine Einfachbindung
m = eine Zahl von 1 bis 40

darstellen,
und
(b) mindestens einen hydrophilen Emulgator der allgemeinen Formel

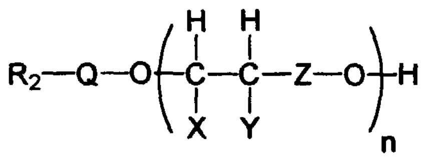

(Emulgator B),

wobei

$R_2$ = $C_{10-30}$-Alkyl
Q = Methylen oder Carbonyl
X = H oder Methyl
Y = H oder Methyl
Z = Methylen oder eine Einfachbindung
n = eine Zahl von 10 bis 400

darstellen, mit der Maßgabe, daß der Quotient n/m wenigstens 1,5 betragen muß, sowie
(c) gegebenenfalls weitere Emulgatoren.

**6.** Stabile multiple Emulsionen nach Anspruch 1, dadurch gekennzeichnet, daß der oder die Emulgatoren A gewählt werden aus der Gruppe der Polyoxyethylenfettalkoholether, insbesondere solcher mit der allgemeinen Formel

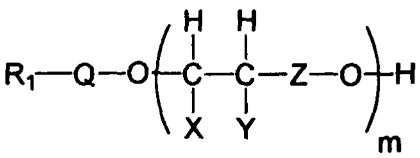

(Emulgator A),

wobei

$R_1$ = $C_{15-25}$-Alkyl
Q = Methylen
X = H
Y = H

Z = eine Einfachbindung
m = eine Zahl von 1 bis 10

bedeuten.

**7.** Stabile multiple Emulsionen nach Anspruch 1, dadurch gekennzeichnet, daß der oder die Emulgatoren B gewählt werden aus der Gruppe der Polyoxyethylenfettalkoholether, insbesondere solcher mit der allgemeinen Formel

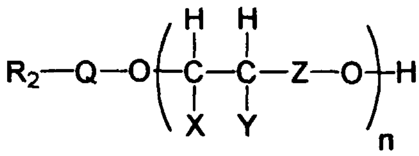

**(Emulgator B),**

wobei

$R_2$ = $C_{15-25}$-Alkyl
Q = Methylen
X = H
Y = H
Z = eine Einfachbindung
n = eine Zahl von 10 bis 400

bedeuten.

**8.** Stabile multiple Emulsionen nach Anspruch 1, dadurch gekennzeichnet, daß der oder die Emulgatoren A gewählt werden aus der Gruppe der Polyoxyethylenfettsäureester, insbesondere solcher mit der allgemeinen Formel

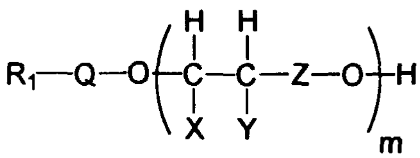

**(Emulgator A),**

wobei

$R_1$ = $C_{15-25}$-Alkyl
Q = Carbonyl
X = H
Y = H
Z = eine Einfachbindung
m = eine Zahl von 1 bis 10

bedeuten.

**9.** Stabile multiple Emulsionen nach Anspruch 1, dadurch gekennzeichnet, daß der oder die Emulgatoren B gewählt werden aus der Gruppe der Polyoxyethylenfettsäureester, insbesondere solcher mit der allgemeinen Formel

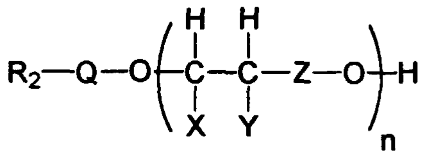

(Emulgator B),

wobei

| | |
|---|---|
| $R_2$ = | $C_{15-25}$-Alkyl |
| Q = | Carbonyl |
| X = | H |
| Y = | H |
| Z = | eine Einfachbindung |
| n = | eine Zahl von 10 bis 400 |

bedeuten.

**10.** Stabile multiple Emulsionen nach Anspruch 1, dadurch gekennzeichnet, daß die Konzentration der Summe von Emulgator A und B zwischen 0,5 und 15,0 %, bezogen auf die gesamte kosmetische oder medizinische Zusammensetzung, beträgt und die molaren Verhältnisse von Emulgator A : Emulgator B von 10 : 1 bis 1 : 10, besonders vorteilhaft 5 : 1 bis 1 : 5, insbesondere 2 : 1 bis 1 : 2, besonders günstig 1,2 : 1 bis 1 : 1,2 betragen.

**11.** Stabile multiple Emulsionen nach Anspruch 1, dadurch gekennzeichnet, daß als Emulgator A Steareth 2 und als Emulgator B Steareth 21 gewählt werden, wobei ein Verhältnis von Emulgator A : Emulgator B von 5 : 1 bis 1 : 5 gewählt wird und die Gesamtmenge an diesem Emulgatorsystem, bezogen auf die Gesamtzusammensetzung, zwischen 1 und 10 % beträgt.

**12.** Stabile multiple Emulsionen nach Anspruch 1, gekennzeichnet durch einen Gehalt an pflanzlichen Ölen mit hohem Linolsäureglyceridanteil, insbesondere Weizenkeimöl. Traubenkernöl, Kukuinosöl, Safloröl, und weiteren Ölen, welche mindestens 5 Gew.-% an Linolsäureglyceriden enthalten, wobei die Emulsionen bevorzugt 0,1 -50 Gew.-% an solchen Ölen oder Abmischungen daraus, bezogen auf das Gesamtgewicht der Zusammensetzung, enthalten.

**13.** Stabile multiple Emulsionen nach Anspruch 1, gekennzeichnet durch einen Gehalt an Stearinsäure und/oder Hydroxyoctacosanyl-hydroxystearat von 0,1 - 10 Gew.-%, insbesondere bei Gewichtsverhältnissen von Stearinsäure und/oder Hydroxyoctacosanylhydroxystearat zueinander von 10 : 1 bis 1 : 10.

**14.** Verfahren zur Herstellung stabiler multipler Emulsionen, dadurch gekennzeichnet, daß

- ein erster, hydrophober Emulgator (Emulgator A), oder mehrere solche Emulgatoren im Gemisch, mit folgenden Eigenschaften:

    - er muß bei der Herstellungstemperatur der Emulsion in der Ölphase der Emulsion zu mehr als 0,01 Gew.-%, insbesondere zu mehr als 5,0 Gew.-% in einem unpolaren Öl löslich sein, wenn ein unpolares Öl verwendet wird, oder
    - er muß bei zu mehr als 0,02 Gew.-%, insbesondere zu mehr als 20,0 Gew.-% in einem polaren Öl löslich sein, wenn ein polares Öl verwendet wird,
    - er darf unterhalb von 50° in Wasser nicht isotrop löslich sein
    - er darf im Temperaturintervall zwischen etwa 50° bis zur Herstellungstemperatur der Emulsion in Wasser isotrop löslich sein
    - er muß im Temperaturintervall zwischen etwa 50° bis zur Herstellungstemperatur der Emulsion primär als W/O-Emulgator fungieren

- der Ölphase einverleibt wird oder werden, und daß
- ein zweiter, hydrophiler Emulgator (Emulgator B), oder mehreren solchen Emulgatoren im Gemisch, mit folgenden Eigenschaften:

- er muß in Wasser isotrop löslich sein,
- er muß im Temperaturintervall zwischen etwa 50° bis zur Herstellungstemperatur der Emulsion eine stabile Löslichkeit aufweisen.

- der Wasserphase einverleibt wird oder werden, und daß Wasser- und Ölphase dann miteinander vereinigt werden.

**15.** Verfahren nach Anspruch 14, dadurch gekennzeichnet, daß die Ölphase mindestens ein polares Öl enthält.

**Claims**

**1.** Stable multiple emulsions of the W/O/W type which comprise at least one polar oil, further comprising an emulsifier mixture consisting of

- a first, hydrophobic emulsifier (emulsifier A), or two or more such emulsifiers in a mixture, having the following properties:
  - it must be soluble, at the preparation temperature of the emulsion, in the oil phase of the emulsion in an amount of more than 0.01% by weight, in particular in an amount of more than 5.0% by weight, in a nonpolar oil, when a nonpolar oil is used, or
  - it must be soluble in the polar oil in an amount of more than 0.02% by weight, in particular in an amount of more than 20.0% by weight,
  - it must not be isotropically soluble in water below 50°
  - it must be isotropically soluble in water in the temperature interval between about 50° up to the preparation temperature of the emulsion
  - it must function primarily as a W/O emulsifier in the temperature interval between about 50° up to the preparation temperature of the emulsion

- and a second, hydrophilic emulsifier (emulsifier B), or two or more such emulsifiers in a mixture, having the following properties:
  - it must be isotropically soluble in water,
  - it must have stable solubility in the temperature interval between about 50° up to the preparation temperature of the emulsion.

**2.** Stable multiple emulsions according to Claim 1, in which the hydrophobic emulsifer(s) A is/are chosen from the group consisting of glycerol ethers, polyglycerol ethers, polyglycerol esters, methyl glucosides, sucrose esters, alkyl polyglycosides, sorbitan esters, sorbitol esters and isosorbide esters.

**3.** Stable multiple emulsions according to Claim 1, in which the hydrophilic emulsifer(s) B is/are chosen from the group consisting of glycerol ethers, polyglycerol ethers, polyglycerol esters, methyl glucosides, sucrose esters, alkyl polyglycosides, sorbitan esters, sorbitol esters and isosorbide esters.

**4.** Stable multiple emulsions according to Claim 1, in which the hydrophobic emulsifier(s) A is/are chosen from the group consisting of sorbitan oleate, sorbitan monostearate, steareth-2, glycerol monostearate, glycerol oleate, polyglycerol-4 isostearate, PEG-7 hydrogenated castor oil, PEG-40 sorbitan monostearate, laureth-4, and the hydrophilic emulsifiers B are chosen from the group consisting of oleth-20, PEG-40 stearate, sucrose cocoate, trilaureth-4 phosphate, gluceth-20, PPG-20 methylglucose ether, polyglycerol-3 stearate, polysorbate-120, sucrose laurate, ceteareth 20.

**5.** Stable multiple emulsions according to Claim 1, comprising

(a) at least one hydrophobic emulsifier of the general formula

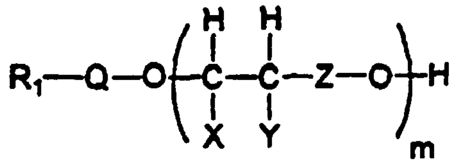

(Emulsifier A).

where

R1 = $C_{10-30}$-alkyl
Q = methylene or carbonyl
X = H or methyl
Y = H or methyl
Z = methylene or a single bond
m = a number from 1 to 40,

and
(b) at least one hydrophilic emulsifier of the general formula

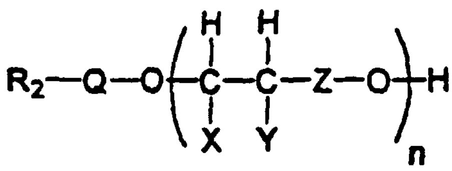

(Emulsifier B).

where

$R_2$ = $C_{10-30}$-alkyl
Q = methylene or carbonyl
X = H or methyl
Y = H or methyl
Z = methylene or a single bond
n = a number from 10 to 400

with the proviso that the quotient n/m must be at least 1.5,
and
(c) optionally further emulsifiers.

6. Stable multiple emulsions according to Claim 1, characterized in that the emulsifier(s) A is/are chosen from the group of polyoxyethylene fatty alcohol ethers, in particular those having the general formula

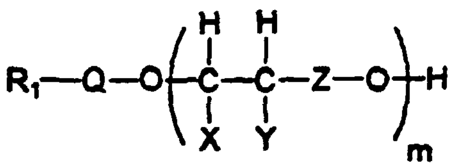

(Emulsifier A).

where

$R_1$ = $C_{15-25}$-alkyl
Q = methylene
X = H

Y = H
Z = a single bond
m = a number from 1 to 10.

**7.** Stable multiple emulsions according to Claim 1, characterized in that the emulsifier(s) B is/are chosen from the group of polyoxyethylene fatty alcohol ethers, in particular those having the general formula

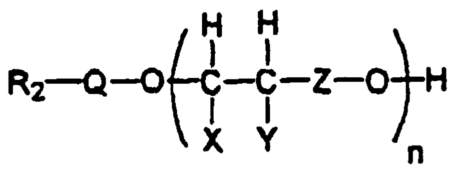

(Emulsifier B).

where

$R_2$ = $C_{15-25}$-alkyl
Q = methylene
X = H
Y = H
Z = a single bond
n = a number from 10 to 400.

**8.** Stable multiple emulsions according to Claim 1, characterized in that the emulsifier(s) A is/are chosen from the group of polyoxyethylene fatty acid esters, in particular those having the general formula

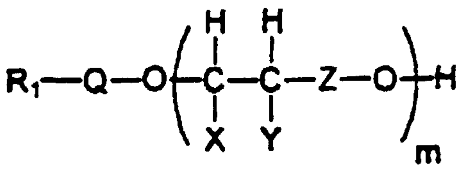

(Emulsifier A).

where

$R_1$ = $C_{15-25}$-alkyl
Q = carbonyl
X = H
Y = H
Z = a single bond
m = a number from 1 to 10.

**9.** Stable multiple emulsions according to Claim 1, characterized in that the emulsifier(s) B is/are chosen from the group of polyoxyethylene fatty acid esters, in particular those having the general formula

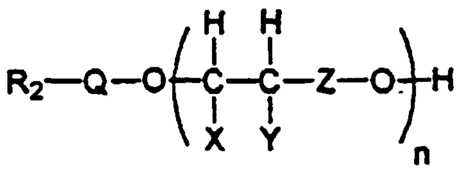

(Emulsifier B).

where

$R_2$ = $C_{15-25}$-alkyl
Q = carbonyl

X = H
Y = H
Z = a single bond
n = a number from 10 to 400.

**10.** Stable multiple emulsions according to Claim 1, characterized in that the concentration of the sum of emulsifier A and B is between 0.5 and 15.0%, based on the overall cosmetic or medicinal composition, and the molar ratios of emulsifier A:emulsifier B are from 10:1 to 1:10, particularly advantageously 5:1 to 1:5, in particular 2:1 to 1:2, particularly favourably 1.2:1 to 1:1.2.

**11.** Stable multiple emulsions according to Claim 1, characterized in that emulsifier A is steareth 2 and emulsifier B is steareth 21, a ratio of emulsifier A:emulsifier B of from 5:1 to 1:5 being chosen, and the total amount of this emulsifier system, based on the total composition, being between 1 and 10%.

**12.** Stable multiple emulsions according to Claim 1, characterized by a content of vegetable oils having a high linoleic acid glyceride content, in particular wheatgerm oil, grapeseed oil, kukui nut oil, safflower oil, and other oils which comprise at least 5% by weight of linoleic acid glycerides, where the emulsions preferably comprise 0.1-50% by weight of such oils or mixtures thereof, based on the total weight of the composition.

**13.** Stable multiple emulsions according to Claim 1, characterized by a content of stearic acid and/or hydroxyoctacosanyl hydroxystearate of 0.1-10% by weight, in particular at weight ratios of stearic acid and/or hydroxyoctacosanyl hydroxystearate to one another of from 10:1 to 1:10.

**14.** Process for the preparation of stable multiple emulsions, characterized in that

- a first, hydrophobic emulsifier (emulsifier A), or two or more such emulsifiers in a mixture, having the following properties:

  - it must be soluble, at the preparation temperature of the emulsion, in the oil phase of the emulsion in an amount of more than 0.01% by weight, in particular in an amount of more than 5.0% by weight, in a nonpolar oil, when a nonpolar oil is used, or
  - it must be soluble in a polar oil in an amount of more than 0.02% by weight, in particular in an amount of more than 20.0% by weight, when a polar oil is used,
  - it must not be isotropically soluble in water below 50°
  - it must be isotropically soluble in water in the temperature interval between about 50° up to the preparation temperature of the emulsion
  - it must function primarily as a W/O emulsifier in the temperature interval between about 50° up to the preparation temperature of the emulsion

- is or are incorporated into the oil phase, and in that
- a second, hydrophilic emulsifier (emulsifier B), or two or more such emulsifiers in a mixture, having the following properties:

  - it must be isotropically soluble in water,
  - it must have stable solubility in the temperature interval between about 50° up to the preparation temperature of the emulsion.

- is or are incorporated into the water phase, and in that

water and oil phase are then combined with one another.

**15.** Process according to Claim 14, characterized in that the oil phase comprises at least one polar oil.

**Revendications**

**1.** Emulsions multiples stables du type E/H/E, comprenant au moins une huile polaire, et comprenant en outre un mélange d'émulsifiants constitué de

- un premier émulsifiant hydrophobe (émulsifiant A), ou plusieurs émulsifiants de ce type en mélange, ayant les propriétés suivantes :

  - il doit être soluble, à la température de préparation de l'émulsion, dans la phase huileuse de l'émulsion, à plus de 0,01% en poids, en particulier à plus de 5,0% en poids, dans une huile non polaire, lorsque l'on utilise une huile non polaire, ou
  - il doit être soluble à plus de 0,02% en poids, en particulier à plus de 20,0% en poids, dans l'huile polaire,
  - il ne doit pas être isotropiquement soluble dans l'eau en dessous de 50°,
  - il ne doit pas être isotropiquement soluble dans l'eau dans l'intervalle de températures compris entre environ 50° et la température de préparation de l'émulsion,
  - il doit fonctionner principalement en tant qu'émulsifiant E/H dans l'intervalle de températures compris entre environ 50° et la température de préparation de l'émulsion,

- et un deuxième émulsifiant hydrophile (émulsifiant B) ou plusieurs émulsifiants de ce type en mélange, ayant les propriétés suivantes :

  - il doit être isotropiquement soluble dans l'eau,
  - il doit présenter une solubilité stable dans l'intervalle de températures compris entre environ 50° et la température de préparation de l'émulsion.

**2.** Emulsions multiples stables selon la revendication 1, dans lesquelles le ou les émulsifiant(s) hydrophobe(s) A est (sont) choisi(s) parmi le groupe constitué d'éthers de glycérol, d'éthers de polyglycérol, d'esters de polyglycérol, de méthylglucosides, d'esters de saccharose, d'alkylpolyglycosides, d'esters de sorbitane, d'esters de sorbitol et d'esters d'isosorbide.

**3.** Emulsions multiples stables selon la revendication 1, dans lesquelles le ou les émulsifiant(s) hydrophobe(s) B est (sont) choisi(s) parmi le groupe constitué d'éthers de glycérol, d'éthers de polyglycérol, d'esters de polyglycérol, de méthylglucosides, d'esters de saccharose, d'alkylpolyglycosides, d'esters de sorbitane, d'esters de sorbitol et d'esters d'isosorbide.

**4.** Emulsions multiples stables selon la revendication 1, dans lesquelles le ou les émulsifiant(s) hydrophobe(s) A est (sont) choisi(s) parmi le groupe constitué de l'oléate de sorbitane, du monostéarate de sorbitane, du stéareth-2, du monostéarate de glycérol, de l'oléate de glycérol, de l'isostéarate de polyglycérol-4, du PEG-7/huile de ricin hydrogénée, du sorbitane monostéarate de PEG/40, du laureth-4, et les émulsifiants hydrophiles B sont choisis parmi le groupe constitué de l'oleth-20, du stéarate de PEG-40, du cocoate de sucrose, du phosphate de trilaureth-4, du gluceth-20, du méthylglucose-éther de PPG-20, du stéarate de polyglycérol-3, du polysorbate-120, du laurate de sucrose et du cétéareth 20.

**5.** Emulsions multiples stables selon la revendication 1, comprenant

(a) au moins un émulsifiant hydrophobe de formule générale

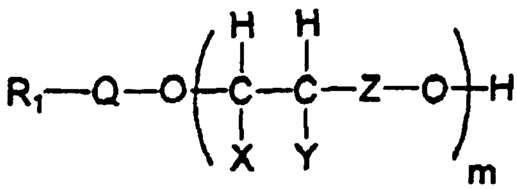

**(Emulsifiant A),**

dans laquelle

$R_1$ = un alkyle en $C_{10-30}$
Q = un méthylène ou un carbonyle
X = H ou un méthyle
Y = H ou un méthyle
Z = un méthylène ou une liaison simple

m = un nombre de 1 à 40

et

(b) au moins un émulsifiant hydrophile de formule générale

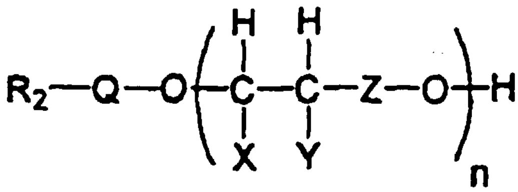

(Emulsifiant B),

dans laquelle

$R_2$ = un alkyle en $C_{10-30}$
Q = un méthylène ou un carbonyle
X = H ou un méthyle
Y = H ou un méthyle
Z = un méthylène ou une liaison simple
n = un nombre de 1 à 400

à condition que le quotient n/m vaille au moins 1,5,
ainsi que
(c) éventuellement d'autres émulsifiants.

**6.** Emulsions multiples stables selon la revendication 1, caractérisées en ce que le ou les émulsifiant(s) A est (sont) choisi(s) parmi le groupe constitué d'éthers d'alcools gras et de polyoxyéthylène, en particulier ceux de formule générale

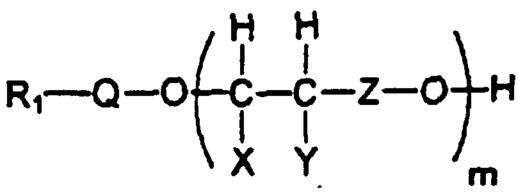

(Emulsifiant A),

dans laquelle

$R_1$ = un alkyle en $C_{15-25}$
Q = un méthylène
X = H
Y = H
Z = une liaison simple
m = un nombre de 1 à 10.

**7.** Emulsions multiples stables selon la revendication 1, caractérisées en ce que le ou les émulsifiant(s) B est (sont) choisi(s) parmi le groupe constitué d'éthers d'alcools gras et de polyoxyéthylène, en particulier ceux de formule générale

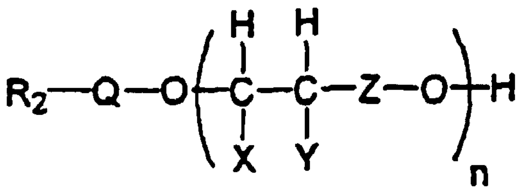

(Emulsifiant B),

dans laquelle

$R_2$ = un alkyle en $C_{15\text{-}25}$
Q = un méthylène
X = H
Y = H
Z = une liaison simple
n = un nombre de 1 à 400.

**8.** Emulsions multiples stables selon la revendication 1, caractérisées en ce que le ou les émulsifiant(s) A est (sont) choisi(s) parmi le groupe constitué d'esters d'acides gras et de polyoxyéthylène, en particulier ceux de formule générale

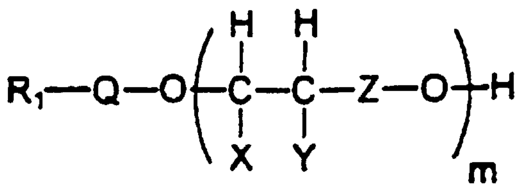

(Emulsifiant A),

dans laquelle

$R_4$ = un alkyle en $C_{15\text{-}25}$
Q = un carbonyle
X = H
Y = H
Z = une liaison simple
m = un nombre de 1 à 10.

**9.** Emulsions multiples stables selon la revendication 1, caractérisées en ce que le ou les émulsifiant(s) B est (sont) choisi(s) parmi le groupe constitué d'esters d'acides gras et de polyoxyéthylène, en particulier ceux de formule générale

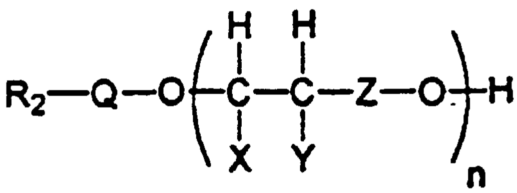

(Emulsifiant B),

dans laquelle

$R_2$ = un alkyle en $C_{15\text{-}25}$
Q = un carbonyle
X = H
Y = H
Z = une liaison simple

n = un nombre de 10 à 400.

**10.** Emulsions multiples stables selon la revendication 1, caractérisées en ce que la concentration de la somme des émulsifiants A et B est comprise entre 0,5 et 15,0%, par rapport à l'ensemble de la composition cosmétique ou médicale, et les rapports molaires émulsifiant A : émulsifiant B sont de 10:1 à 1:10, particulièrement avantageusement de 5:1 à 1:5, en particulier de 2:1 à 1:2, de manière particulièrement favorable de 1,2:1 à 1:1,2.

**11.** Emulsions multiples stables selon la revendication 1, caractérisées en ce que le stéareth 2 est choisi en tant qu'émulsifiant A et le stéareth 21 en tant qu'émulsifiant B, en choisissant un rapport émulsifiant A : émulsifiant B de 5:1 à 1:5 et la quantité totale de ce système émulsifiant étant de 1 à 10%, par rapport à l'ensemble de la composition.

**12.** Emulsions multiples stables selon la revendication 1, caractérisées par une teneur en huiles végétales à forte proportion de glycérides de l'acide linoléique, en particulier l'huile de germes de blé, l'huile de pépins de raisin, l'huile de noix de kukui, l'huile de carthame et d'autres huiles comprenant au moins 5% en poids de glycérides de l'acide linoléique, les émulsions comprenant de préférence de 0,1 à 50% en poids de telles huiles ou de leurs mélanges, par rapport au poids total de la composition.

**13.** Emulsions multiples stables selon la revendication 1, caractérisées par une teneur en acide stéarique et/ou hydroxystéarate d'hydroxyoctacosanyle de 0,1 à 10% en poids, en particulier à des rapports pondéraux entre l'acide stéarique et/ou l'hydroxystéarate d'hydroxyoctacosanyle de 10:1 à 1:10.

**14.** Procédé de préparation d'émulsions multiples stables, caractérisé en ce que

- un premier émulsifiant hydrophobe (émulsifiant A), ou plusieurs émulsifiants de ce type en mélange, ayant les propriétés suivantes :

  - il doit être soluble, à la température de préparation de l'émulsion, dans la phase huileuse de l'émulsion, à plus de 0,01% en poids, en particulier à plus de 5,0% en poids, dans une huile non polaire, lorsque l'on utilise une huile non polaire, ou
  - il doit être soluble à plus de 0,02% en poids, en particulier à plus de 20,0% en poids, dans une huile polaire, lorsqu'on utilise une huile polaire,
  - il ne doit pas être isotropiquement soluble dans l'eau en dessous de 50°,
  - il ne doit pas être isotropiquement soluble dans l'eau dans l'intervalle de températures compris entre environ 50° et la température de préparation de l'émulsion,
  - il doit fonctionner principalement en tant qu'émulsifiant E/H dans l'intervalle de températures compris entre environ 50° et la température de préparation de l'émulsion,

- est ou sont incorporé(s) dans la phase huileuse, et en ce que
- et un deuxième émulsifiant hydrophile (émulsifiant B) ou plusieurs émulsifiants de ce type en mélange, ayant les propriétés suivantes :

  - il doit être isotropiquement soluble dans l'eau,
  - il doit présenter une solubilité stable dans l'intervalle de températures compris entre environ 50° et la température de préparation de l'émulsion

- et ou sont incorporé(s) dans la phase aqueuse, et en ce que la phase aqueuse et la phase huileuse sont alors combinées l'une à l'autre.

**15.** Procédé selon la revendication 14, caractérisé en ce que la phase huileuse comprend au moins une huile polaire.